(19) Europäisches Patentamt
European Patent Office
Office européen des brevets

(11) Publication number: 0 397 914 A1

# (12) EUROPEAN PATENT APPLICATION

(21) Application number: 89114805.8

(22) Date of filing: 10.08.89

(30) Priority: 17.05.89 JP 121593/89
19.05.89 JP 126119/89

(43) Date of publication of application:
22.11.90 Bulletin 90/47

(84) Designated Contracting States:
BE CH DE FR GB IT LI NL SE

(51) Int. Cl.5: A61K 35/78, A61K 31/35

(71) Applicant: MITSUI NORIN CO., LTD.
1-20, 3-chome, Nihonbashimuromachi
Chuo-ku
Tokyo(JP)

(72) Inventor: Hara, Yukihiko
2-7, 2-chome, Minamisurugadai
Fujieda-shi Shizuoka-ken(JP)
Inventor: Watanabe, Mayumi
7-41, 7-chome, Chiyoda
Shizuoka-shi Shizuoka-ken(JP)
Inventor: Ishigami, Tadashi
No. 9256-13, Motoshimada
Shimada-shi Shizuoka-ken(JP)
Inventor: Shimamura, Tadakatsu
4-4, 1-chome, Nishihara Shibuya-ku
Tokyo(JP)
Inventor: Sakaguchi, Genji
1-1-913, 1-chome, Wakamatsudai
Sakai-shi Osaka(JP)

(74) Representative: Türk, Gille, Hrabal
Brucknerstrasse 20
D-4000 Düsseldorf 13(DE)

## (54) Antitoxins for bacterial exotoxins.

(57) The present invention provides an antitoxin mainly comprising of tea polyphenol(s). The antitoxin counteracts bacterial exotoxins and is usable for the prevention or treatment of an interoinfection without causing any adverse reactions.

EP 0 397 914 A1

# ANTITOXINS FOR BACTERIAL EXOTOXINS

## BACKGROUND OF THE INVENTION

The present invention relates to antitoxins for bacterial exotoxins. In particular, the present invention relates to an antitoxins capable of preventing or treating intestinal toxicosis caused by bacteria such as Clostridium botulinum, Vibrio cholerae, Vibrio parahaemolyticus and Staphylococcus aureus.

In developing countries where insufficient sanitation and undernourishment prevailes, peaple suffer from cholera of a high lethality. Not only in these countries but even in advanced countries, sitotoxism associated with Vibrio parahaemolyticus or Staphylococcus aureus frequently occurs due to improper handling of foods and drinks.

Someone of the present inventors previously reported that a tea extract has an antibacterial effect towards these enteroinfectious bacteria. The sitotoxism due to these pathogenic bacteria is caused when the toxins produced in the foods are taken or by the toxins produced in the course of proliferation of bacteria in the intestinal tracts.

Clostridium botulinum which is a pathogenic bacterium that causes botulism is widely distributed in the soil and it might contaminate various kinds of food. The botulism is caused by a toxin formed in the foods by Clostridum botulinum. It is important, therefore, to prevent foods or drinks from the contamination by the pathogenic bacteria or the proliferation thereof. Further sitotoxism due to these bacteria can be prevented or the symptoms can be relieved by converting a toxin into a non-toxic substance. Chemicals having such an antitoxic effect without harmful side effects have been eagerly wanted.

## SUMMARY OF THE INVENTION

After intensive investigations to obtain a substance having the above-described medicinal effect from natural resources, the inventor has found that such a substance is contained in tea. The present invention has been completed on the basis of this finding.

## DETAILED DESCRIPTION OF THE INVENTION

The present invention provides antitoxins mainly comprised of tea polyphenols which counteract bacterial exotoxins.

The tea polyphenols which are the main component of the antitoxins of the present invention include tea catechins of the following general formula I and theaflavins of the following general formula II:

General formula 1:

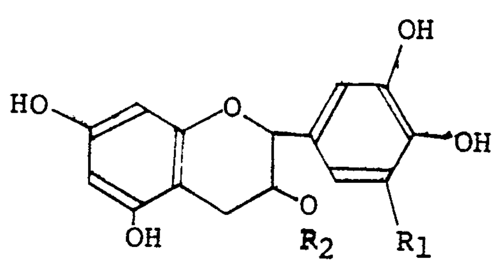

..... I

wherein $R_1$ represents H or OH and $R_2$ represents H or

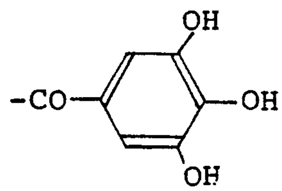

Examples of the catechins include the following compounds:

(-) epicatechin of the general formula 1 wherein $R_1$ represents H and $R_2$ represents H,

(-) epigallocatechin of the general formula I wherein $R_1$ represents OH and $R_2$ represents H,

(-) epicatechin gallate of the general formula I wherein $R_1$ represents H and $R_2$ represents

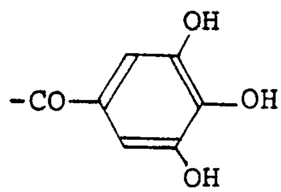

(-) epigallocatechin gallate of the general formula I wherein $R_1$ represents OH and $R_2$ represents

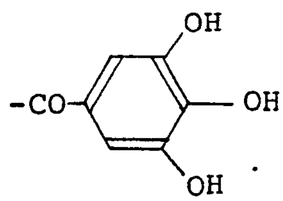

General formula II:

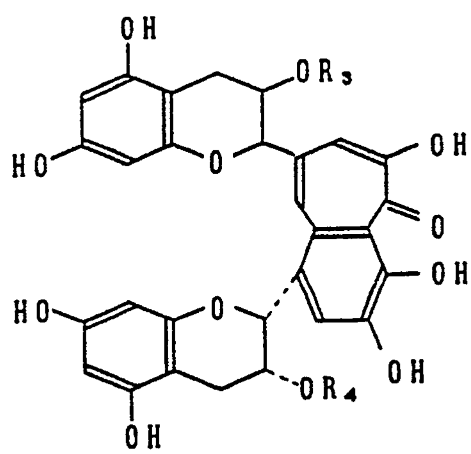

wherein $R_3$ and $R_4$ may be the same or different from each other and each represent H or

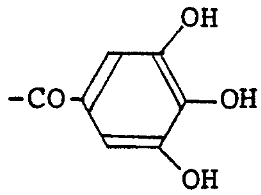

Examples of the theaflavins include the following compounds:

• free theaflavin of the general formula II wherein $R_3$ represents H and $R_4$ represents H,

• theaflavin monogallate A of the general formula II wherein $R_3$ represents

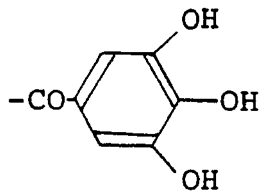

and $R_4$ represents H,

• theaflavin monogallate B of the general formula II wherein $R_3$ represents H and $R_4$ represents

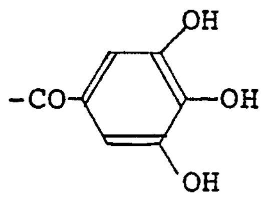

• theaflavin digallate of the general formula II wherein $R_3$ represents

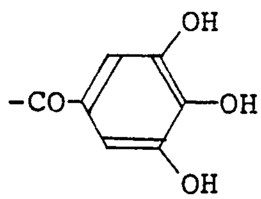

and $R_4$ represents

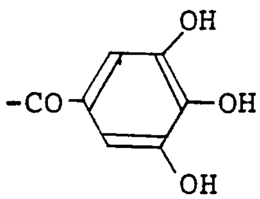

The tea polyphenols can be produced from tea leaves. Processes for producing them and compositions of them are described in, for example, Japanese Patent Kokai Koho Nos. 219384/1984, 13780/1985 and 130285/1986.

The exotoxins to be treated according to the present invention are those formed by bacteria such as Clostridium botulinum, Vibrio cholerae, Vibrio parahaemolyticus and Staphylococcus aureus.

The antitoxins of the present invention are added to a food in order to prevent the sitotoxism due to the

above-described bacteria in the following manner: the above-described tea polyphenol (active ingredient) is added to the food directly or after dissolving it in water or in an alcohol. The concentration of the antitoxins is preferably from 0.005 to 1%, more preferably from 0.005 to 0.1%, in the food. When the antitoxin of the present invention is to be administered to a human body, the tea polyphenol (active ingredient) is diluted with a suitable filler and orally administered in the form of a powder, tablets or liquid. However, since the patients suffering from the bacterial exotoxin are dehydrated due to diarrhea, it is desirable to incorporate the antitoxin of the present invention in an oral rehydration solution having the following composition;

| Formulation | |
|---|---|
| common salt | 2 to 5 g |
| potassium chloride | 1 to 3 g |
| calcium lactate | 100 to 300 mg |
| vitamin C | 50 to 100 mg |
| glucose | 10 to 50 g |
| antitoxin of the present invention | 10 mg to 1 g |

A powdery mixture of these components is dissolved in 1 ℓ of sterilized water and a necessary dose of the solution is orally administered to the patient. The amount of the antitoxin of the present invention is preferably from 10 mg to 1 g, more preferably from 30 to 500 mg.

The antitoxin of the present invention for the exotoxins exhibits no adverse reaction, since it mainly comprises natural ingredients of tea which is ordinarily taken in a large amount. It exhibits a strong specific effect on exotoxins produced by Clostridium botulinum, Vibrio cholerae, Vibrio parahaemolyticus and Staphylococcus aureus. The antitoxin of the present invention is, therefore, quite effective for the prevention or treatment of an interoinfection.

The following examples will further illustrate the present invention.

Experimental Example

Epigallocatechin gallate was orally administered to male ICR mice (6 weeks old). $LD_{50}$ of them after one week was 2314 mg/kg. In another case, epigallocatechin gallate was administered to male ICR mice (6 weeks old) by intraperitoneal injection. $LD_{50}$ of them after one week was 150 mg/kg.

Example 1

The antitoxic activities were determined as follows: the hemolytic activities of α-toxin, hemolysin of Vibrio parahaemolyticus and Vibrio cholerae were determined by using erythrocytes of rabbits as the target cells by hemolysis velocity method. The antitoxic effects of tea polyphenols were evaluated from their rates of inhibition of the hemolysis activity of the toxin. The inhibition rates of 33 µg/ml of tea polyphenols for α-toxin (1 µg/ml, hemolytic activity: 0.22/min), hemolysins of Vibrio parahaemolyticus (4.9 µg/ml, hemolytic activity: 0.049/min) and Vibrio cholerae (hemolytic activity: 0.39/min) were as follows:

| | |
|---|---|
| epigallocatechin gallate | 100% |
| epicatechin gallate | 70 to 100% |
| epigallocatechin | 30 to 70% |
| epicatechin | 30 to 100% |
| (+) catechin | 5 to 20% |
| free theaflavin | 100% |
| theaflavin monogallate A | 100% |
| theaflavin monogallate B | 100% |
| theaflavin digallate | 100% |

It is apparent from the above-described results that the catechin gallates and theaflavins have a marked antitoxic effect. It seems possible that these tea polyphenols work on both the toxin and erythrocytes to inhibit hemolysis.

Example 2

The antitoxic activities of tea polyphenols against botulinum toxins were determined as follows: a solution of M toxin or S toxin of A type toxins produced by Clostridium botulinum was mixed with a solution of a tea polyphenol. The mixture was left to stand at 37° C for 120 min and administered to mice by intravenous injection. The lethal time (min) was counted and the toxic activity was calculated. The antitoxic effect of the tea polyphenol was evaluated from the reduction of the toxic activity.

The results are shown in Table 1. The tea polyphenols used were free theaflavin, theaflavin monogallate A, theaflavin monogallate B, theaflavin digallate, nonfractionated crude theaflavin containing at least 60% of these components as the main components, Polyphenone TF (a product of Mitsui Norin Co., Ltd.) which was derived from a black tea extract and contained at least 20% of polyphenols in total, Polyphenone BT (a product of Mitsui Norin Co., Ltd.) which was a crude polyphenol preparation and Polyphenone 100 (a product of Mitsui Norin Co., Ltd.) which was a polyphenol preparation derived from a green tea extract and containing at least 80% of epigallocatechin gallate, epicatechin gallate, epigallocatechin and epicatechin as main components.

It is apparent from the following table that the tea polyphenols which remarkably neutralized the toxic activity of the toxin produced by Clostridium botulinum are useful as an effective antitoxin.

Table 1

| Conc. of tea polyphenol | Remaining toxic activity (%) | | | | | |
|---|---|---|---|---|---|---|
| | M toxin (10 µg/ml) | | | S toxin (10 µg/ml) | | |
| | 0.1 mg/ml | 1.0 mg/ml | 10 mg/ml | 0.1 mg/ml | 1.0 mg/ml | 10 mg/ml |
| Free theaflavin | 9.9 | 7.6 | - | 4.4 | 6.1 | - |
| Theaflavin monogallate A | <0.7 | 0.7 | - | 2.4 | 20.5 | - |
| Theaflavin monogallate B | <0.8 | 1.0 | - | <0.4 | 12.4 | - |
| Theaflavin digallate | 33.8 | <1.0 | - | <0.5 | <0.5 | - |
| Crude theaflavin | 33.6 | 12.3 | - | 1.3 | 2.9 | - |
| Polyphenone TF | 27.3 | 4.3 | - | 0.7 | 0.09 | - |
| Polyphenone BT | - | 0.57 | <0.1 | - | <0.1 | <0.07 |
| Polyphenone 100 | - | 80.4 | 6.2 | - | 7.6 | 4.1 |

**Claims**

1. An antitoxin mainly comprising a tea polyphenol which counteracts bacterial exotoxins.

2. An antitoxin according to Claim 1 wherein the tea polyphenol is at least one substance selected from the group consisting of epigallocatechin gallate, epicatechin gallate, epigallocatechin, epicatechin, (+) catechin, free theaflavin, theaflavin monogallate A, theaflavin monogallate B and theaflavin digallate.

3. An antitoxin according to Claim 1 wherein the bacterial exotoxin is produced by any of Clostridium botulinum, Vibrio cholerae, Vibrio parahaemolyticus and Staphylococcus aureus.

European Patent Office

# EUROPEAN SEARCH REPORT

Application Number

EP 89 11 4805

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | BIOLOGICAL ABSTRACTS, vol. 88, 1989, abstract no. 60772, Philadelphia, PA, US; Y. HARA et al.: "The fate of Clostridium botulinum spores inoculated into tea drinks (Studies on anti-bacterial effects of tea polyphenols: Part I)", & J. JPN. SOC. FOOD. SCI. TECHNOL. 36(5): 375-379 * Abstract * --- | 1-3 | A 61 K 35/78<br>A 61 K 31/35 |
| A | DE-A-1 060 087 (CHEMISCHE WERKE ALBERT) ----- | | |
| | | | **TECHNICAL FIELDS SEARCHED (Int. Cl.5)** |
| | | | A 61 K |

The present search report has been drawn up for all claims

| Place of search | Date of completion of the search | Examiner |
|---|---|---|
| THE HAGUE | 13-06-1990 | REMPP G.L.E. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document